# EUROPEAN PATENT APPLICATION

(11) **EP 2 044 970 A1**
(43) Date of publication of application: **08.04.2009**
(21) Application number: 08165861.9
(22) Date of filing: 03.10.2008
(51) Int. Cl.: A61M 25/06, A61M 5/32

(54) **Closed system safety device for venous and arterial cannulation**

(30) Priority: 04.10.2007 IT RC20070015
(71) Applicant: Medi-Line Srl, 89068 Frazione San Gregorio, RC (IT)
(72) Inventor: Cuzzucoli, Antonino, 89100 Reggio Calabria (IT)
(74) Representative: Jorio, Paolo

(57) **Abstract**

A venous and arterial cannulation device comprising a sliding structure (2), a pusher element (3) having a supporting surface (4) suitable for being engaged by a finger of the operator's hand and positioned to close a further end (2a) of the sliding structure (2), a catheter holder element (5) fixed in a removable manner to close one end (2b) of the sliding structure (2) and a needle holder element (6) inserted inside the sliding structure (2) where it is free to slide between an advanced position, in which a needle (8) is inserted in a catheter (14) from which the tip protrudes, and a retracted position in which the needle (8) is completely inside the sliding structure (2). The needle holder element (6) comprises one or more gripping portions (9) extending outside the sliding structure (2) and suitable for being engaged by other fingers of said operator's hand for moving said needle holder element (6) from the advanced position to the retracted position.

## Description

The present invention relates to a venous and arterial cannulation device.

Although currently the most advanced venous cannulation devices have solved the problems relating to protection of the needle when the latter is extracted and separated from the catheter, they nevertheless still have drawbacks, above all as regards manual handling, which at times can negatively affect the cannulation operation.

In fact, said devices do not always allow the operator to perform the operation with one single hand and, in any case, correct cannulation of the vessel still depends to a large extent on the ability of the operator and the physical conditions of the patient.

For a better understanding of the problems described above, it should be specified that the main phases of the cannulation procedure are: a) insertion of the needle/catheter device in the vein or artery, b) advancing of the catheter and needle unit in the vein or artery and c) extraction of the needle from the system. In phase a) the needle and catheter are inserted in the blood vessel until the blood flows towards the outside, shown in the display chamber, thus confirming that the needle and catheter are inside the blood vessel. In phase b) the operator has to keep the needle still, in position, advancing only the catheter which, once free of the needle, (phase c), will adapt perfectly to the morphology of the blood vessel. With the current devices, it is not always easy to control the advance of the catheter, hence the catheter often "overshoots" the vein. This problem is caused by the needle which, if advanced farther than necessary during insertion of the catheter, can damage not only the catheter but also the vessel or even pass through it. Said occurrence will either require a fresh cannulation operation using a new device or, at worst, negatively affect the use of that access point for a varying period of time or even cause phlebitis.

Lastly, it should be highlighted that the need to use two hands for the cannulation operation makes the operation very difficult, not to say impossible, in emergency situations in which the patient is in a state of confusion.

The subject of the present invention is a venous and arterial cannulation device, the basic characteristics of which are described in claim 1, and the preferred and/or auxiliary characteristics of which are described in the claims 2-5.

For a better understanding of the present invention, a preferred embodiment is now described purely by way of nonlimiting example and with reference to the accompanying figures, in which:
figure 1 is a longitudinal section of the device subject of the present invention in a first configuration; and
figure 2 is a longitudinal section of the device subject of the present invention in a second configuration.

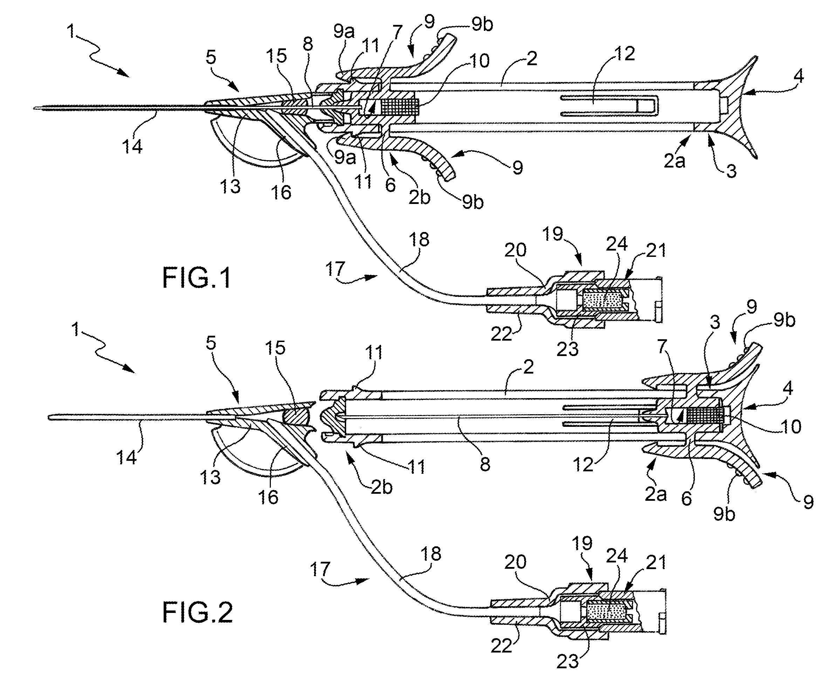

In figures 1 and 2, 1 indicates overall the device subject of the present invention.

The device 1 comprises a cylindrical shaped sliding structure 2 made of transparent material, a pusher element 3 having an ergonomic supporting surface 4 for a finger of the operator's hand and positioned to close a first end 2a of the sliding structure 2, a catheter holder element 5 fixed in a removable manner and closing a second end 2b of the sliding structure 2 and a needle holder element 6 inserted inside the sliding structure 2 where it is free to move as described below.

The needle holder element 6 comprises a display chamber 7 directly connected to a needle 8 and two dovetail fins 9 extending, during use, outside the sliding structure 2 via two longitudinal slots obtained on opposite sides in the sliding structure 2 itself. Each of the two dovetail fins 9 has a locking end 9a to ensure the stability of the configuration illustrated in figure 1, and a gripping surface 9b shaped so as to be engaged in a stable manner by the operator's finger. The display chamber 7 houses an absorbing element 10 to prevent any leaks of liquid from the display chamber 7.

The sliding structure 2 comprises two stop wedges 11 extending on opposite sides in the vicinity of its second end 2b and snap-engaging with the two locking ends 9a, and two flexible tab portions 12 also snap-engaging with a lower end of the display chamber 7 to ensure the stability of the configuration illustrated in figure 2.

The catheter holder element 5 comprises a supporting cone 13 with bayonet connection to the end 2a of the sliding structure 2, a catheter 14 fixed to the supporting cone 13, a plug 15 housed in the supporting cone and made of thermoplastic material or silicone so that it can be crossed by the needle 8 and re-closed once the needle 8 is removed in order to prevent the reflux of liquid outside the catheter holder element 5, and an inlet appendix 16 facing directly onto the supporting cone 13 for the connection of a lateral infusion branch 17. The lateral infusion branch 17 comprises a tube 18 and a water-repellent anti-reflux valve 19 to ensure that there is no risk of accidental introduction of air into the bloodstream.

In particular, the water-repellent anti-reflux valve 19 comprises a watertight containment structure 20 having at one end a female luer fitting 21 for luer-lock connections or syringe cone and at a second end a tapered fitting 22 for coupling with the tube 18. The containment structure 20 houses a hollow thermoplastic rubber element 23 which acts as a stop valve and a water-repellent microporous filter 24 which occupies the cavity of the hollow rubber element 23.

During the cannulation procedure the blood flows towards the valve along the tube 18 pushing all the residual air inside the tube 18. The air compressed by the blood is expelled to the outside via the water-repellent microporous filter 24, while the thermoplastic rubber element 23, which is in its closing position, guarantees that the blood is retained inside the containment structure 20. The connection of an administration device via the female luer fitting 21 compresses the thermoplastic rubber element 23 thus permitting passage of the fluid without leaks of liquid and without air.

During use, starting from the configuration illustrated in figure 1, the operator grips with one hand the device 1 and manoeuvres it to prick the blood vessel until blood appears in the display chamber 7. At this point, the operator modifies his grip on the device by placing his thumb on the supporting surface 4 with his index and middle fingers engaging the dovetail fins 9 in the manner of a "syringe grip". In this position the operator pushes with his thumb on the surface 4 keeping still his index and middle fingers which engage the two fins 9. In this way the sliding structure 2 and the catheter holder element 5 advance while the needle holder element 6 remains stationary inside the sliding structure 2 as far as stroke end where the flexible tabs 12 snap-engage the end of the display chamber 7 thus producing the configuration illustrated in figure 2. In this way the catheter is inserted in the blood vessel without any risk. In fact, unlike the devices of the known art, in the device of the present invention once the blood is visible in the display chamber 7 as a result of the needle accessing the vein, advance of the needle is stopped, for example, by placing the index and middle fingers on the patient's skin, and exercising a slight pressure on the surface 4 with the thumb of the same hand, so that only the catheter advances, gradually adapting perfectly to the vessel.

Once the configuration of figure 2 has been obtained, the needle 8 is completely housed in the sliding structure 2 and therefore does not pose any danger, and the operator can always release, with the same hand, the catheter holder element 5 from the sliding structure 2.

As is evident from the above description, the advantages of the device subject of the present invention, when compared with the same devices of the known art, are that it can be handled by the operator with one single hand and that it ensures correct insertion of the catheter without having to rely exclusively on the experience and professional skill of the operator.

## Claims

1. Venous and arterial cannulation device comprising a sliding structure (2), a catheter holder element (5) fixed in a removable manner and closing one end (2b) of the sliding structure (2) and a needle holder element (6) inserted inside the sliding structure (2) where it is free to move between an advanced position in which a needle (8) is inserted in a catheter (14) with the tip protruding from it and a retracted position in which said needle (8) is completely inside said sliding structure (2); said device being **characterised in that** said needle holder element (6) comprises one or more gripping portions (9) extending outside said sliding structure (2) and suitable for being engaged by one or more fingers of the operator's hand, and **in that** it comprises a pusher element (3) positioned to close a further end (2a) of the sliding structure (2) and having a supporting surface (4) suitable for being engaged by a finger of said operator's hand for moving said needle holder element (6) from said advanced position to said retracted position.

2. Device as claimed in claim 1, **characterised in that** said gripping portions comprise two dovetail fins (9) extending during use outside the sliding structure (2) through two longitudinal slots obtained on opposite sides in the sliding structure (2).

3. Device as claimed in claim 2, **characterised in that** each of the two dovetail fins (9) comprises a locking end (9a) to ensure the stability of the advanced position, and a gripping surface (9b) shaped so as to be engaged in a stable manner by the operator's finger.

4. Device as claimed in claim 3, **characterised in that** it comprises a lateral infusion branch (17) comprising a tube (18) and a water-repellent anti-reflux valve (19) to eliminate the risk of accidentally introducing air into the bloodstream.

5. Device as claimed in claim 3, **characterised in that** said water-repellent anti-reflux valve (19) comprises a watertight containment structure (20) having at one first end a female luer fitting (21) for luer-lock connections or syringe cone and at a second end a tapered fitting (22) for coupling with the tube (18); the containment structure (20) houses a hollow thermoplastic rubber element (23) which acts as a stop valve and a water-repellent microporous filter (24) which occupies the cavity of the hollow rubber element (23).
